# EUROPÄISCHE PATENTSCHRIFT

(11) **EP 0 485 783 B1**
(45) Veröffentlichungstag und Bekanntmachung des Hinweises auf die Patenterteilung: **11.01.1995**
(21) Anmeldenummer: 91118217.8
(22) Anmeldetag: 25.10.1991
(51) Int. Cl.: C25B 3/02

(54) **Verfahren zur Herstellung von Epoxiden**
Process for producing epoxy compounds
Procédé de préparation d'époxydes

(30) Priorität: 16.11.1990 DE 4036511
(43) Veröffentlichungstag der Anmeldung: 20.05.1992
(73) Patentinhaber: BASF Aktiengesellschaft, 67063 Ludwigshafen (DE)
(72) Erfinder: Hermeling, Dieter, Dr., W-6710 Frankenthal (DE)

(56) Entgegenhaltungen:
- CH-A- 642 960
- CHEMICAL ABSTRACTS, Band 93, Nr. 6, September 1980, Seite 502, Zusammenfassung Nr. 122677s, Columbus, Ohio, US; & JP-A-80 50 474
- ELECTROCHIMICA ACTA, Band 26, Nr .6, Juni 1981, Seiten 763-770, Pergamon Press Ltd, Oxford, GB; R. CELDRAN et al.: "The oxidation of allyl alcohol on Au-electrode in acidic electrolytes"

## Beschreibung

Die vorliegende Erfindung betrifft ein Verfahren zur Herstellung von Epoxiden durch elektrochemische Oxidation von Allylalkoholen in Gegenwart von Alkanolen und eines Hilfselektrolyten sowie neue Epoxide.

Aus J. Chem. Soc. 1393-1395 (1948) ist die Umsetzung von Chlor mit Allylalkohol im sauren Milieu zu 2,3-Dichlorpropanol bekannt. Unter vergleichbaren Bedingungen sind nach Houben-Weyl Bd. 5/4, 4. Auflage, S. 55 bis 58 und 133 bis 141 Bromhydrine durch Umsetzung von Allylalkoholen mit Brom oder Brom der Oxidationsstufe (+1) zugänglich.

Die Umsetzung von Olefinen mit Chlor oder Chlor der Oxidationsstufe (+1), bzw. Brom oder Brom der Oxidationsstufe (+1) in Gegenwart von Alkohol führt dagegen nach Houben-Weyl 5/3, 4. Auflage, S. 775 ff und Houben-Weyl 5/4, 4. Auflage, S. 141-146 zu Halogenhydrinethern.

Die Synthese von 3-substituierten-3-Hydroxy-1,2-epoxiden aus 3-substituierten-Alk-2-en-1-olen gelingt z.B. nach Tetrahedron Lett. 27, 4987-4990 (1986), Chem. Lett. 645-646 (1988) nur über einen separaten Isomerisierungsschritt der primär synthetisierten 3-substituierten 1-Hydroxy-2,3-epoxide, oder die Isomerisierung erfolgt wie in Helv. Chim. Acta 54, 1822-1845 (1971) beschrieben, bereits auf der Stufe der Allylalkohole.

Die CH-A-642 960 beschreibt die Oxidation von bestimmten Allylalkoholen mittels Brom, wonach aus den erhallenen Zwischenprodukten durch Behandlung mit kalilange 3-Alkoxy-1,2-epoxide erhältlich sind.

Eine Direktsynthese von 3-Hydroxy- oder 3-Alkoxy-1,2-epoxiden aus 1-Hydroxyalk-2-enen, wobei das Sauerstoffatom im Edukt das Epoxidsauerstoffatom im Produkt darstellt, ist bisher nicht bekannt.

Der Erfindung lag daher die Aufgabe zugrunde, eine solche Direktsynthese zu entwickeln.

Demgemäß wurde ein neues Verfahren zur Herstellung von Epoxiden der allgemeinen Formel I 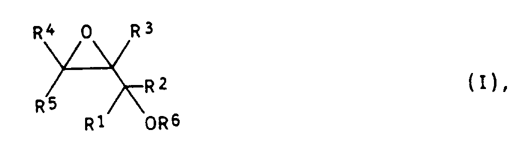
in der die Substituenten
- R¹, R², R³, R⁴, R⁵: unabhängig voneinander Wasserstoff, C₁- bis C₂₀-Alkyl, C₃- bis C₁₂-Cycloalkyl, C₄- bis C₂₀-Cycloalkyl-alkyl, C₁- bis C₂₀-Hydroxyalkyl, einen Heterocyclus, gegebenenfalls durch C₁- bis C₈-Alkyl, C₁- bis C₈-Alkoxy, Halogen, C₁- bis C₄-Halogenalkyl, C₁- bis C₄-Halogenalkoxy, Phenyl, Phenoxy, Halogenphenyl, Halogenphenoxy, Carboxy, C₂- bis C₈- Alkoxycarbonyl oder Cyano substituiertes Aryl oder C₇- bis C₂₀-Arylalkyl, oder R¹ und R² oder R¹ und R³ oder R¹ und R⁴ oder R³ und R⁴ oder R⁴ und R⁵ gemeinsam eine gegebenenfalls durch C₁- bis C₈-Alkyl, C₁- bis C₈-Alkoxy und oder Halogen ein- bis zweifach substituierte (CH₂)ₙ-Gruppe, in der n für 1 bis 10 und
- R⁶: C₁- bis C₈-Alkyl oder Benzyl
bedeuten, gefunden, welches dadurch gekennzeichnet ist, daß man Allylalkohole der allgemeinen Formel II 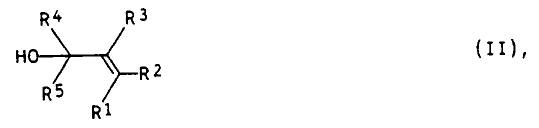
in der R¹, R², R³, R⁴ und R⁵ die obengenannte Bedeutung haben, in Gegenwart eines Alkanols der allgemeinen Formel III

R⁶-OH (III),

in der R⁶ die obengenannte Bedeutung hat,
in Gegenwart eines Hilfselektrolyten elektrochemisch oxidiert,
sowie neue Verbindungen der allgemeinen Formel IV 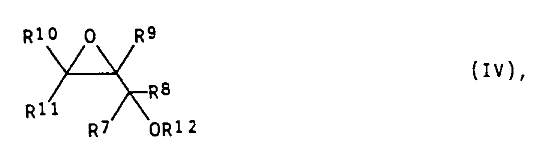
in der die Substituenten folgende Bedeutung haben:
- R⁷, R⁸, R⁹, R¹⁰, R¹¹: Wasserstoff, C₁- bis C₂₀-Alkyl, C₃- bis C₁₂-Cycloalkyl, C₄- bis C₂₀-Cycloalkyl-alkyl, C₁- bis C₂₀-Hydroxyalkyl, einen Heterocyclus, gegebenenfalls durch C₁- bis C₈-Alkyl, C₁- bis C₈-Alkoxy, Halogen, C₁- bis C₄-Halogenalkyl, C₁- bis C₄-Halogenalkoxy, Phenyl, Phenoxy, Halogenphenyl, Halogenphenoxy, Carboxy, C₂- bis C₈- Alkoxycarbonyl oder Cyano substituiertes Aryl oder C₇- bis C₂₀-Arylalkyl, oder R⁷ und R⁸ oder R⁷ und R⁹ oder R⁷ und R¹⁰ oder R⁹ und R¹⁰ oder R¹⁰ und R¹¹ gemeinsam eine gegebenenfalls durch C₁- bis C₈-Alkyl, C₁- bis C₈-Alkoxy und oder Halogen ein- bis zweifach substituierte (CH₂)ₙ-Gruppe, in der n für 1 bis 10 steht
- R¹²: C₁- bis C₈-Alkyl oder Benzyl
bedeuten,
mit der Maßgabe, daß R¹⁰ nicht Wasserstoff, Methyl, Ethyl, Propyl oder Cyclohexyl bedeutet, wenn R⁷, R⁸, R⁹, R¹¹ Wasserstoff ist, daß R⁷ nicht Methyl bedeutet; wenn R⁸ Methyl oder Phenyl und R⁹, R¹⁰, R¹¹ Wasserstoff ist, daß R⁷ nicht Methyl bedeutet, wenn R⁸, R⁹, R¹⁰, R¹¹ Wasserstoff und R¹² Ethyl ist, daß R⁷ nicht Phenyl oder Hexyl bedeutet, wenn R⁸, R⁹, R¹⁰, R¹¹ Wasserstoff ist, daß R⁹ nicht Methyl bedeutet, wenn R⁷, R⁸, R¹⁰, R¹¹ Wasserstoff ist, daß R¹⁰, R¹¹ nicht Methyl bedeutet, wenn R⁷, R⁸, R⁹ Wasserstoff ist, daß R¹² nicht Methyl ist, wenn R⁸ Phenyl ist und R⁷, R⁹, R¹⁰, R¹¹ Wasserstoff ist, und daß R⁷ und R¹⁰ gemeinsam nicht ein unsubstituiertes Cycloalkyl bedeuten, wenn R⁸, R⁹, R¹¹ Wasserstoff ist.

Als Ausgangsverbindungen zur Herstellung der erfindungsgemäßen Epoxide kommen prinzipiell alle Allylalkohole der allgemeinen Formel II in Betracht, die unter den Elektrolysebedingungen inerte Substituenten tragen.

Als Substituenten R¹ bis R⁶ und Index n in den Formeln I bis III kommen unabhängig voneinander für das Verfahren folgende Bedeutungen in Betracht:
- R¹, R², R³, R⁴, R⁵: - Wasserstoff,
- unverzweigtes oder verzweigtes C₁- bis C₂₀-Alkyl, vorzugsweise unverzweigtes oder verzweigtes C₁- bis C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl,
- C₄- bis C₂₀-Cycloalkyl-alkyl, bevorzugt C₄- bis C₈-Cycloalkyl-alkyl, wie Cyclopentyl-alkyl, 2-Cyclopentyl-ethyl, 1-Cyclopentyl-ethyl, Cycloalkyl-methyl, 1-Cyclohexyl-ethyl und 2-Cyclohexyl-ethyl,
- unverzweigtes oder verzweigtes C₁- bis C₂₀-Hydroxyalkyl, bevorzugt unverzweigtes oder verzweigtes C₁- bis C₈-Hydroxyalkyl, wie Hydroxymethyl, 1-Hydroxyethyl, 2-Hydroxyethyl, 1-Hydroxypropyl, 2-Hydroxypropyl und 3-Hydroxypropyl,
- ein Heterocyclus wie ein drei- bis 12-gliedrigen Heterocyclus, vorzugsweise ein 3- bis 8-gliedrigen Heterocyclus wie Oxiranyl, Tetrahydrofuran-2-yl, Tetrahydrofuran-3-yl, Tetrahydropyran-2-yl, Tetrahydropyran-3-yl, Tetrahydropyran-4-yl, 1,3-Dioxolan-2-yl, 1,3-Dioxolan-4-yl, Dioxan-2-yl, Pyrrolidin-2-yl, N-Methylpyrrolidin-2-yl, N-Methylpyrrolidin-3-yl, Pyrrolidin-3-yl, Piperidin-2-yl, Piperidin-3-yl, Piperidin-4-yl, Morpholin-2-yl und Morpholin-3-yl,
- Aryl, wie Phenyl, 1-Naphthyl und 2-Naphthyl, bevorzugt Phenyl,
- ein- bis dreifach durch C₁- bis C₈-Alkyl substituiertes Aryl, bevorzugt ein- bis dreifach durch C₁- bis C₄-Alkyl substituiertes Phenyl, wie 2-Methylphenyl, 4-Methylphenyl, 2-Ethylphenyl, 4-Ethylphenyl, 3-Methylphenyl, 2,4-Dimethylphenyl, 3,4-Dimethylphenyl und 3,4,5-Trimethylphenyl,
- ein- bis dreifach durch C₁- bis C₈-Alkoxy substituiertes Aryl, bevorzugt ein- bis dreifach durch C₁- bis C₄-Alkoxy substituiertes Phenyl, wie 2-Methoxyphenyl, 2-Ethoxyphenyl, 3-Methoxyphenyl, 4-Methoxyphenyl, 3-Ethoxyphenyl, 4-Ethoxyphenyl, 3,4-Dimethoxyphenyl und 3,4,5-Trimethoxyphenyl,
- ein- bis dreifach durch C₁- bis C₄-Halogenalkyl substituiertes Aryl, bevorzugt ein- bis dreifach durch C₁- bis C₂-Fluor- und Chloralkyl Phenyl, besonders bevorzugt ein- bis dreifach durch Trifluormethyl und Trichlorethyl, substituiertes Phenyl, wie 4-Trifluormethylphenyl und 4-Trichlormethylphenyl,
- ein- bis dreifach durch C₁- bis C₄-Halogenalkoxy substituiertes Aryl, bevorzugt ein- bis dreifach durch C₁- bis C₂-Fluor- und Chloralkoxy Phenyl, besonders bevorzugt ein- bis dreifach durch Trifluormethoxy und Trichlormethoxy substituiertes Phenyl, wie Trifluormethoxyphenyl,
- ein- bis dreifach durch Halogen substituiertes Aryl, bevorzugt ein- bis dreifach durch Fluor, Chlor oder Brom substituiertes Phenyl, wie 4-Chlorphenyl, 3,4-Dichlorphenyl, 4-Bromphenyl, 4-Fluorphenyl und 4-Fluor-3-chlorphenyl,
- ein- bis dreifach durch Halogenphenyl substituiertes Aryl, bevorzugt ein- bis dreifach durch Fluor- und/oder Chlorphenyl substituiertes Phenyl wie (4-Chlorphenyl)-phenyl,
- ein- bis dreifach durch Halogenphenoxy substituiertes Aryl, bevorzugt ein- bis dreifach durch Fluor- und/oder Chlorphenyl substituiertes Phenyl, wie (4-Fluorphenoxy)-phenyl,
- ein- bis dreifach durch Carboxy substituiertes Aryl, bevorzugt ein- bis dreifach durch Carboxy substituiertes Phenyl, wie 2-Carboxyphenyl, 3-Carboxyphenyl und 4-Carboxyphenyl,
- ein- bis dreifach durch C₂- bis C₈-Alkoxycarbonyl substituiertes Aryl, bevorzugt ein- bis dreifach durch C₂- bis C₄-Alkoxycarbonyl substituiertes Phenyl, wie 4-Methoxycarbonylphenyl, 4-Ethoxycarbonylphenyl, 2-Methoxycarbonylphenyl, 2-Ethoxycarbonylphenyl und 3-Methoxycarbonylphenyl,
- ein- bis dreifach durch Cyano substituiertes Aryl, bevorzugt ein- bis dreifach durch Cyano substituiertes Phenyl, wie 2-Cyanophenyl, 3-Cyanophenyl und 4-Cyanophenyl,
- C₇- bis C₂₀-Arylalkyl, bevorzugt C₇- bis C₁₂-Arylalkyl wie Benzyl, Phenylethyl, Phenylpropyl und Phenylisopropyl,
- im Arylteil ein- bis dreifach durch Halogen substituiertes C₇- bis C₂₀-Aryl-alkyl, bevorzugt im Phenylteil ein- bis dreifach durch Fluor oder Chlor C₇- bis C₁₀-Phenyl-alkyl, wie 4-Fluorbenzyl, 4-Chlorbenzyl, 2,4-Dichlorbenzyl und 3,4-Dichlorbenzyl,
- im Arylteil ein- bis dreifach durch C₁- bis C₈-Alkyl substituiertes C₇- bis C₂₀-Aryl-alkyl, bevorzugt im Phenylteil ein- bis dreifach durch C₁- bis C₄-Alkyl substituiertes C₇- bis C₁₀-Phenyl-alkyl, besonders bevorzugt im Phenylteil ein- bis dreifach durch C₁- bis C₂-Alkyl substituiertes C₇- bis C₁₀-Phenylalkyl, wie 4-Methylphenyl, 4-Ethylbenzyl und 4-Methylphenethyl,
- im Arylteil ein- bis dreifach durch C₁- bis C₈-Alkoxy substituiertes C₇- bis C₂₀-Aryl-alkyl, bevorzugt im Phenylteil ein- bis dreifach durch C₁- bis C₄-Alkoxy C₇- bis C₁₀-Phenylalkyl, besonders bevorzugt im Phenylteil ein- bis dreifach durch C₁- bis C₂-Alkoxy substituiertes C₇- bis C₁₀-Phenylalkyl, wie 4-Methoxybenzyl, 4-Ethoxybenzyl und 4-Methoxyphenethyl,
- im Arylteil ein- bis dreifach durch C₁- bis C₄-Halogenalkyl, substituiertes C₇- bis C₂₀-Arylalkyl, bevorzugt im Phenylteil ein- bis dreifach durch C₁- bis C₂-Fluor- und Chloralkyl substituiertes C₇- bis C₁₀-Phenylalkyl, besonders bevorzugt im Phenylteil ein- bis dreifach durch Trifluormethyl und Trichlormethyl substituiertes C₇-C₁₀-Phenylalkyl, wie 4-Trifluormethylbenzyl und 4-Trichlormethylbenzyl,
- im Arylteil ein- bis drei Fach durch C₁- bis C₄-Halogenalkoxy substituiertes C₇- bis C₂₀-Arylalkyl, bevorzugt im Phenylteil ein- bis dreifach durch C₁- bis C₂-Halogenalkyl substituiertes C₇- bis C₁₀-Phenyl-alkyl, besonders bevorzugt ein- bis dreifach durch Trifluormethyl und Trichlormethyl substituiertes C₇- bis C₁₀-Phenylalkyl, wie 4-Trifluormethoxybenzyl und 4-Trichlormethoxybenzyl,
- ein- bis dreifach durch Halogenphenyl substituiertes C₇- C₂₀-Arylalkyl, bevorzugt ein- bis dreifach durch Fluor- und/oder Chlorphenyl substituiertes C₇- bis C₁₂-Phenylalkyl, wie 4-Chlorphenethyl und 4-Fluorphenethyl,
- ein- bis dreifach durch Halogenphenoxy substituiertes C₇- C₂₀-Arylalkyl, bevorzugt ein- bis dreifach durch Fluor- und/oder Chlorphenyl substituiertes C₇- bis C₁₂-Phenylalkyl, wie 2-Chlorphenoxymethyl und 4-Chlorphenoxymethyl,
- ein- bis dreifach durch Carboxy substituiertes C₇- C₂₀-Arylalkyl, bevorzugt ein- bis dreifach durch Carboxy substituiertes C₇- bis C₁₂-Phenylalkyl, wie 4-Carboxybenzyl, 4-Carboxyphenethyl, 2-Carboxybenzyl und 4-Carboxyphenylethyl,
- ein- bis dreifach durch C₂- bis C₈-Alkoxycarbonyl substituiertes C₇- C₂₀-Arylalkyl, bevorzugt ein- bis dreifach durch C₂- bis C₄-Alkoxycarbonyl substituiertes C₇- bis C₁₂-Phenylalkyl, wie 4-Methoxycarbonylbenzyl, 2-Methoxycarbonylbenzyl, 4-Ethoxycarbonylbenzyl und 2-Ethoxycarbonylbenzyl,
- ein- bis dreifach durch Cyano substituiertes C₇- C₂₀-Arylalkyl, bevorzugt ein- bis dreifach durch Cyano substituiertes C₇- bis C₁₂-Phenylalkyl, wie 2-Cyanobenzyl, 4-Cyanobenzyl, 2-Cyanophenethyl und 4-Cyanophenethyl,
- durch ein, zwei oder drei Phenylgruppen substituiertes Phenyl, wie 2-(Phenyl)-phenyl, 3-(Phenyl)-phenyl, 4-(Phenyl)-phenyl und 3,4-(Diphenyl)-phenyl,
- durch ein, zwei oder drei Phenoxygruppen substituiertes Phenyl, wie 4-Phenoxyphenyl und 2-Phenoxyphenyl,
- durch Halogen und C₁- bis C₄-Alkyl zwei- oder dreifach substituiertes Phenyl, wie 2-Methyl-4-chlorphenyl und 3-Methyl-4-fluorphenyl,
- durch Halogen und C₁- bis C₄-Alkoxy zwei- oder dreifach substituiertes Phenyl, wie 3-Chlor-4-methoxyphenyl,
- durch Halogen und C₁- bis C₄-Halogenalkyl zwei- oder dreifach substituiertes Phenyl, wie 2-Chlor-4-trifluormethylphenyl,
- durch Halogen und Phenoxy zwei- oder dreifach substituiertes Phenyl, wie 3-Chlor-4-phenoxyphenyl,
- durch C₁- bis C₄-Alkyl und C₁- bis C₄-Alkoxy zwei- oder dreifach substituiertes Phenyl, wie 2-Methyl-4-methoxyphenyl,
- durch C₁- bis C₄-Alkyl und C₁- bis C₄-Halogenalkyl zwei- oder dreifach substituiertes Phenyl, wie 3-Methyl-4-trichlormethylphenyl,
- durch C₁- bis C₄-Alkyl und Phenoxy zwei- oder dreifach substituiertes Phenyl, wie 2-Methyl-4-phenoxyphenyl,
- durch C₁- bis C₄-Alkoxy und C₁- bis C₄-Halogenalkyl zwei- oder dreifach substituiertes Phenyl, wie 3-Trifluormethyl-4-methoxyphenyl,
- durch C₁- bis C₄-Alkoxy und Phenoxy zwei- oder dreifach substituiertes Phenyl, wie 3-Methoxy-4-phenoxyphenyl,
- durch C₁- bis C₄-Halogenalkyl und Phenoxy zwei- oder dreifach substituiertes Phenyl, wie 3-Trifluormethyl-4-phenoxyphenyl,
- durch Halogen, C₁- bis C₄-Alkyl und C₁- bis C₄-Alkoxy dreifach substituiertes Phenyl, wie 2-Chlor-3-tert.butyl-4-methoxyphenyl,
- durch Halogen, C₁- bis C₄-Alkyl und C₁- bis C₄-Halogenalkyl dreifach substituiertes Phenyl, wie 2-Methyl-3-chlor-4-trifluormethylphenyl,
- durch Halogen, C₁- bis C₄-Alkyl und Phenoxy dreifach substituiertes Phenyl, wie 4-Chlor-2-ethyl-3-phenoxyphenyl,
- durch Halogen, C₁- bis C₄-Alkoxy und C₁- bis C₄-Halogenalkyl dreifach substituiertes Phenyl, wie 3-Chlor-4-methoxy-3-trifluormethyl,
- durch Halogen, C₁- bis C₄-Alkoxy und Phenoxy dreifach substituiertes Phenyl, wie 2-Fluor-4-ethoxy-3-phenoxyphenyl,
- durch Halogen, C₁- bis C₄-Halogenalkyl und Phenoxy dreifach substituiertes Phenyl, wie 4-Fluor-3-trifluormethyl-2-phenoxyphenyl,
- durch C₁- bis C₄-Alkyl, C₁- bis C₄-Alkoxy und C₁- bis C₄-Halogenalkyl dreifach substituiertes Phenyl, wie 4-Methyl-3-methoxy-2-trichlormethylphenyl,
- durch C₁- bis C₄-Alkyl, C₁- bis C₄-Alkoxy und Phenoxy dreifach substituiertes Phenyl, wie 4-Methyl-3-ethoxy-2-phenoxyphenyl,
- durch C₁- bis C₄-Alkyl, C₁- bis C₄-Halogenalkyl und Phenoxy dreifach substituiertes Phenyl, wie 2-Methyl-4-trifluormethyl-3-phenoxyphenyl,
- durch C₁- bis C₄-Alkoxy, C₁- bis C₄-Halogenalkyl und Phenoxy dreifach substituiertes Phenyl, wie 4-Methoxy-2-trichlormethyl-3-phenoxyphenyl,
R¹ und R² oder R¹ und R³ oder R¹ und R⁴ oder R³ und R⁴ oder R⁴ und R⁵ gemeinsam
- (CH₂)ₙ, wie CH₂, (CH₂)₂, (CH₂)₃, (CH₂)₄, (CH₂)₅ und (CH₂)₆, bevorzugt (CH₂)₃, (CH₂)₄, (CH₂)₅, (CH₂)₆, besonders bevorzugt (CH₂)₃ und (CH₂)₄,

- n: - 1 bis 6, vorzugsweise 3 bis 6, besonders bevorzugt 3 und 4,
- R⁶: - C₁- bis C₈-Alkyl, insbesondere C₁- bis C₄-Alkyl wie Methyl, Ethyl, n-Propyl und n-Butyl,
- Benzyl.

Ein- bis dreifach substituiert bedeutet ein-, zwei- oder dreifach substituiert.

Die elektrochemische Oxidation läßt sich bei (-20) bis 150°C, vorzugsweise bei 0 bis
100°C im Druckbereich von 0,01 bis 50 bar, vorzugsweise 0,1 bis 5 bar, besonders bevorzugt bei Atmosphärendruck (Normaldruck) durchführen:
Um eine für die Elektrolyse ausreichende Leitfähigkeit des Elektrolyten zu gewährleisten, wird der Elektrolysemischung ein vorzugsweise halogenhaltiger Hilfselektrolyt zugefügt. Als Hilfselektrolyte kommen beispielsweise elementares Halogen, Alkylhalogenide und Halogenwasserstoff und Halogenide, bevorzugt Iodide und Bromide wie Ammoniumhalogenide z.B. Ammoniumbromid, Ammoniumjodid und Tetrabutylammoniumjodid und besonders bevorzugt Metallhalogenide wie Natriumbromid, Natriumjodid, Kaliumbromid und Kaliumjodid in Betracht.
Reicht die Leitfähigkeit der Halogenverbindung alleine nicht aus, können auch andere in der Elektrochemie gebräuchliche Hilfselektrolyte wie z.B. Tetrafluoroborate, Tetraalkylammoniumsalze wie Tetraethylammoniumbenzolsulfonat, Perchlorate wie LiClO₄, Methylate wie Natriummethylat, Hydroxide wie NaOH oder KOH zugesetzt werden.
Die Zusammensetzung der Elektrolyten kann in weiten Grenzen gewählt werden. Diese enthalten bevorzugt
1 bis 49, vorzugsweise 5 bis 30 Gew.% Allylalkohol II
50 bis 98,9, vorzugsweise 70 bis 95 Gew.% Alkanol R⁶-OH und
0,1 bis 5, vorzugsweise 0,5 bis 3 Gew.% Hilfselektrolyt
(katalytische Mengen).

Der Elektrolyt kann 0 bis 10 Gew.% Wasser enthalten.

Vorzugsweise nimmt man die elektrochemische Oxidation bei Stromdichten von 0,5 bis 25 A/dm² und bei Temperaturen von 0 bis 100, insbesondere 10 bis 50°C vor. Die Reaktion kann bei vermindertem, erhöhtem und vorzugsweise bei Normaldruck (Atmosphärendruck) in an sich üblichen Elektrolysezellen, vorzugsweise in ungeteilten Durchflußzellen durchgeführt werden.

Als Anodenmaterialien eignen sich beispielsweise Edelmetalle wie Platin oder Oxide wie Ruthenium- oder Chromoxid oder RuOₓ/TiOₓ-Mischoxide und bevorzugt Graphit.

Als Kathodenmaterialien kommen in der Regel Eisen, Stahl, Nickel und Edelmetalle wie Platin und bevorzugt Graphit in Betracht.

Die Aufarbeitung erfolgt in an sich bekannter Weise; vorzugsweise werden die erfindungsgemäßen Verbindungen destillativ aufgearbeitet.

In den erfindungsgemäßen Verbindungen der allgemeinen Formel IV haben die Substituenten folgende Bedeutung:
- R⁷, R⁸, R⁹, R¹⁰, R¹¹: unabhängig voneinander
- Wasserstoff,
- unverzweigtes oder verzweigtes C₁- bis C₂₀-Alkyl, vorzugsweise unverzweigtes oder verzweigtes C₁- bis C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl,
- C₄- bis C₂₀-Cycloalkyl-alkyl, bevorzugt C₄- bis C₈-Cycloalkyl-alkyl, wie Cyclopentyl-alkyl, 2-Cyclopentyl-ethyl, 1-Cyclopentyl-ethyl, Cycloalkyl-methyl, 1-Cyclohexyl-ethyl und 2-Cyclohexyl-ethyl,
- unverzweigtes oder verzweigtes C₁- bis C₂₀-Hydroxyalkyl, bevorzugt unverzweigtes oder verzweigtes C₁- bis C₈-Hydroxyalkyl, wie Hydroxymethyl, 1-Hydroxyethyl, 2-Hydroxyethyl, 1-Hydroxypropyl, 2-Hydroxypropyl und 3-Hydroxypropyl,
- ein Heterocyclus wie ein drei- bis 12-gliedriger Heterocyclus, vorzugsweise ein 3- bis 8-gliedriger Heterocyclus wie Oxiranyl, Tetrahydrofuran-2-yl, Tetrahydrofuran-3-yl, Tetrahydropyran-2-yl, Tetrahydropyran-3-yl, Tetrahydropyran-4-yl, 1,3-Dioxolan-2-yl, 1,3-Dioxolan-4-yl, Dioxan-2-yl, Pyrrolidin-2-yl, N-Methylpyrrolidin-2-yl, N-Methylpyrrolidin-3-yl, Pyrrolidin-3-yl, Piperidin-2-yl, Piperidin-3-yl, Piperidin-4-yl, Morpholin-2-yl und Morpholin-3-yl,
- Aryl, wie Phenyl, 1-Naphthyl und 2-Naphthyl, bevorzugt Phenyl,
- ein- bis dreifach durch C₁- bis C₈-Alkyl substituiertes Aryl, bevorzugt ein- bis dreifach durch C₁- bis C₄-Alkyl substituiertes Phenyl, wie 2-Methylphenyl, 4-Methylphenyl, 2-Ethylphenyl, 4-Ethylphenyl, 3-Methylphenyl, 2,4-Dimethylphenyl, 3,4-Dimethylphenyl und 3,4,5-Trimethylphenyl,
- ein- bis dreifach durch C₁- bis C₈-Alkoxy substituiertes Aryl, bevorzugt ein- bis dreifach durch C₁- bis C₄-Alkoxy substituiertes Phenyl, wie 2-Methoxyphenyl, 2-Ethoxyphenyl, 3-Methoxyphenyl, 4-Methoxyphenyl, 3-Ethoxyphenyl, 4-Ethoxyphenyl, 3,4-Dimethoxyphenyl und 3,4,5-Trimethoxyphenyl,
- ein- bis dreifach durch C₁- bis C₄-Halogenalkyl substituiertes Aryl, bevorzugt ein- bis dreifach durch C₁- bis C₂-Fluor- und Chloralkyl Phenyl, besonders bevorzugt ein- bis dreifach durch Trifluormethyl und Trichlorethyl, substituiertes Phenyl, wie 4-Trifluormethylphenyl und 4-Trichlormethylphenyl,
- ein- bis dreifach durch C₁- bis C₄-Halogenalkoxy substituiertes Aryl, bevorzugt ein- bis dreifach durch C₁- bis C₂-Fluor- und Chloralkoxy Phenyl, besonders bevorzugt ein- bis dreifach durch Trifluormethoxy und Trichlormethoxy substituiertes Phenyl, wie Trifluormethoxyphenyl,
- ein- bis dreifach durch Halogen substituiertes Aryl, bevorzugt ein- bis dreifach durch Fluor, Chlor oder Brom substituiertes Phenyl, wie 4-Chlorphenyl, 3,4-Dichlorphenyl, 4-Bromphenyl, 4-Fluorphenyl und 4-Fluor-3-chlorphenyl,
- ein- bis dreifach durch Halogenphenyl substituiertes Aryl, bevorzugt ein- bis dreifach durch Fluor- und/oder Chlorphenyl substituiertes Phenyl wie (4-Chlorphenyl)-phenyl,
- ein- bis dreifach durch Halogenphenoxy substituiertes Aryl, bevorzugt ein- bis dreifach durch Fluor- und/oder Chlorphenyl substituiertes Phenyl, wie (4-Fluorphenoxy)-phenyl,
- ein- bis dreifach durch Carboxy substituiertes Aryl, bevorzugt ein- bis dreifach durch Carboxy substituiertes Phenyl, wie 2-Carboxyphenyl, 3-Carboxyphenyl und 4-Carboxyphenyl,
- ein- bis dreifach durch C₂- bis C₈-Alkoxycarbonyl substituiertes Aryl, bevorzugt ein- bis dreifach durch C₂- bis C₄-Alkoxycarbonyl substituiertes Phenyl, wie 4-Methoxycarbonylphenyl, 4-Ethoxycarbonylphenyl, 2-Methoxycarbonylphenyl, 2-Ethoxycarbonylphenyl und 3-Methoxycarbonylphenyl,
- ein- bis dreifach durch Cyano substituiertes Aryl, bevorzugt ein- bis dreifach durch Cyano substituiertes Phenyl, wie 2-Cyanophenyl, 3-Cyanophenyl und 4-Cyanophenyl,
- C₇- bis C₂₀-Arylalkyl, bevorzugt C₇- bis C₁₂-Arylalkyl wie Benzyl, Phenylethyl, Phenylpropyl und Phenylisopropyl,
- im Arylteil ein- bis dreifach durch Halogen substituiertes C₇- bis C₂₀-Aryl-alkyl, bevorzugt im Phenylteil ein- bis dreifach durch Fluor oder Chlor C₇- bis C₁₀-Phenyl-alkyl, wie 4-Fluorbenzyl, 4-Chlorbenzyl, 2,4-Dichlorbenzyl und 3,4-Dichlorbenzyl,
- im Arylteil ein- bis dreifach durch C₁- bis C₈-Alkyl substituiertes C₇- bis C₂₀-Aryl-alkyl, bevorzugt im Phenylteil ein- bis dreifach durch C₁- bis C₄-Alkyl substituiertes C₇- bis C₁₀-Phenyl-alkyl, besonders bevorzugt im Phenylteil ein- bis dreifach durch C₁- bis C₂-Alkyl substituiertes C₇- bis C₁₀-Phenylalkyl, wie 4-Methylphenyl, 4-Ethylbenzyl und 4-Methylphenethyl,
- im Arylteil ein- bis dreifach durch C₁- bis C₈-Alkoxy substituiertes C₇- bis C₂₀-Aryl-alkyl, bevorzugt im Phenylteil ein- bis dreifach durch C₁- bis C₄-Alkoxy C₇- bis C₁₀-Phenylalkyl, besonders bevorzugt im Phenylteil ein- bis dreifach durch C₁- bis C₂-Alkoxy substituiertes C₇- bis C₁₀-Phenylalkyl, wie 4-Methoxybenzyl, 4-Ethoxybenzyl und 4-Methoxyphenethyl,
- im Arylteil ein- bis dreifach durch C₁- bis C₄-Halogenalkyl, substituiertes C₇- bis C₂₀-Arylalkyl, bevorzugt im Phenylteil ein- bis dreifach durch C₁- bis C₂-Fluor- und Chloralkyl substituiertes C₇- bis C₁₀-Phenylalkyl, besonders bevorzugt im Phenylteil ein- bis dreifach durch Trifluormethyl und Trichlormethyl substituiertes C₇-C₁₀-Phenylalkyl, wie 4-Trifluormethylbenzyl und 4-Trichlormethylbenzyl,
- im Arylteil ein- bis dreifach durch C₁- bis C₄-Halogenalkoxy substituiertes C₇- bis C₂₀-Arylalkyl, bevorzugt im Phenylteil ein- bis dreifach durch C₁- bis C₂-Halogenalkyl substituiertes C₇- bis C₁₀-Phenyl-alkyl, besonders bevorzugt ein- bis dreifach durch Trifluormethyl und Trichlormethyl substituiertes C₇- bis C₁₀-Phenylalkyl, wie 4-Trifluormethoxybenzyl und 4-Trichlormethoxybenzyl,
- ein- bis dreifach durch Halogenphenyl substituiertes C₇- C₂₀-Arylalkyl, bevorzugt ein- bis dreifach durch Fluor- und/oder Chlorphenyl substituiertes C₇- bis C₁₂-Phenylalkyl, wie 4-Chlorphenethyl und 4-Fluorphenethyl,
- ein- bis dreifach durch Halogenphenoxy substituiertes C₇- C₂₀-Arylalkyl, bevorzugt ein- bis dreifach durch Fluor- und/oder Chlorphenyl substituiertes C₇- bis C₁₂-Phenylalkyl, wie 2-Chlorphenoxymethyl und 4-Chlorphenoxymethyl,
- ein- bis dreifach durch Carboxy substituiertes C₇- C₂₀-Arylalkyl, bevorzugt ein- bis dreifach durch Carboxy substituiertes C₇- bis C₁₂-Phenylalkyl, wie 4-Carboxybenzyl, 4-Carboxyphenethyl, 2-Carboxybenzyl und 4-Carboxyphenylethyl,
- ein- bis dreifach durch C₂- bis C₈-Alkoxycarbonyl substituiertes C₇- C₂₀-Arylalkyl, bevorzugt ein- bis dreifach durch C₂- bis C₄-Alkoxycarbonyl substituiertes C₇- bis C₁₂-Phenylalkyl, wie 4-Methoxycarbonylbenzyl, 2-Methoxycarbonylbenzyl, 4-Ethoxycarbonylbenzyl und 2-Ethoxycarbonylbenzyl,
- ein- bis dreifach durch Cyano substituiertes C₇- C₂₀-Arylalkyl, bevorzugt ein- bis dreifach durch Cyano substituiertes C₇- bis C₁₂-Phenylalkyl, wie 2-Cyanobenzyl, 4-Cyanobenzyl, 2-Cyanophenethyl und 4-Cyanophenethyl,
- durch ein, zwei oder drei Phenylgruppen substituiertes Phenyl, wie 2-(Phenyl)-phenyl, 3-(Phenyl)-phenyl, 4-(Phenyl)-phenyl und 3,4-(Diphenyl)-phenyl,
- durch ein, zwei oder drei Phenoxygruppen substituiertes Phenyl, wie 4-Phenoxyphenyl und 2-Phenoxyphenyl,
- durch Halogen und C₁- bis C₄-Alkyl zwei- oder dreifach substituiertes Phenyl, wie 2-Methyl-4-chlorphenyl und 3-Methyl-4-fluorphenyl,
- durch Halogen und C₁- bis C₄-Alkoxy zwei- oder dreifach substituiertes Phenyl, wie 3-Chlor-4-methoxyphenyl,
- durch Halogen und C₁- bis C₄-Halogenalkyl zwei- oder dreifach substituiertes Phenyl, wie 2-Chlor-4-trifluormethylphenyl,
- durch Halogen und Phenoxy zwei- oder dreifach substituiertes Phenyl, wie 3-Chlor-4-phenoxyphenyl,
- durch C₁- bis C₄-Alkyl und C₁- bis C₄-Alkoxy zwei- oder dreifach substituiertes Phenyl, wie 2-Methyl-4-methoxyphenyl,
- durch C₁- bis C₄-Alkyl und C₁- bis C₄-Halogenalkyl zwei- oder dreifach substituiertes Phenyl, wie 3-Methyl-4-trichlormethylphenyl,
- durch C₁- bis C₄-Alkyl und Phenoxy zwei- oder dreifach substituiertes Phenyl, wie 2-Methyl-4-phenoxyphenyl,
- durch C₁- bis C₄-Alkoxy und C₁- bis C₄-Halogenalkyl zwei- oder dreifach substituiertes Phenyl, wie 3-Trifluormethyl-4-methoxyphenyl,
- durch C₁- bis C₄-Alkoxy und Phenoxy zwei- oder dreifach substituiertes Phenyl, wie 3-Methoxy-4-phenoxyphenyl,
- durch C₁- bis C₄-Halogenalkyl und Phenoxy zwei- oder dreifach substituiertes Phenyl, wie 3-Trifluormethyl-4-phenoxyphenyl,
- durch Halogen, C₁- bis C₄-Alkyl und C₁- bis C₄-Alkoxy dreifach substituiertes Phenyl, wie 2-Chlor-3-tert.butyl-4-methoxyphenyl,
- durch Halogen, C₁- bis C₄-Alkyl und C₁- bis C₄-Halogenalkyl dreifach substituiertes Phenyl, wie 2-Methyl-3-chlor-4-trifluormethylphenyl,
- durch Halogen, C₁- bis C₄-Alkyl und Phenoxy dreifach substituiertes Phenyl, wie 4-Chlor-2-ethyl-3-phenoxyphenyl,
- durch Halogen, C₁- bis C₄-Alkoxy und C₁- bis C₄-Halogenalkyl dreifach substituiertes Phenyl, wie 3-Chlor-4-methoxy-3-trifluormethyl,
- durch Halogen, C₁- bis C₄-Alkoxy und Phenoxy dreifach substituiertes Phenyl, wie 2-Fluor-4-ethoxy-3-phenoxyphenyl,
- durch Halogen, C₁- bis C₄-Halogenalkyl und Phenoxy dreifach substituiertes Phenyl, wie 4-Fluor-3-trifluormethyl-2-phenoxyphenyl,
- durch C₁- bis C₄-Alkyl, C₁- bis C₄-Alkoxy und C₁- bis C₄-Halogenalkyl dreifach substituiertes Phenyl, wie 4-Methyl-3-methoxy-2-trichlormethylphenyl,
- durch C₁- bis C₄-Alkyl, C₁- bis C₄-Alkoxy und Phenoxy dreifach substituiertes Phenyl, wie 4-Methyl-3-ethoxy-2-phenoxyphenyl,
- durch C₁- bis C₄-Alkyl, C₁- bis C₄-Halogenalkyl und Phenoxy dreifach substituiertes Phenyl, wie 2-Methyl-4-trifluormethyl-3-phenoxyphenyl,
- durch C₁- bis C₄-Alkoxy, C₁- bis C₄-Halogenalkyl und Phenoxy dreifach substituiertes Phenyl, wie 4-Methoxy-2-trichlormethyl-3-phenoxyphenyl,

R⁷ und R⁸ oder R⁷ und R⁹ oder R⁷ und R¹⁰ oder R⁹ und R¹⁰ oder R¹⁰ und R¹¹ gemeinsam
- (CH₂)ₙ, wie CH₂, (CH₂)₂, (CH₂)₃, (CH₂)₄, (CH₂)₅ und (CH₂)₆, bevorzugt (CH₂)₃, (CH₂)₄, (CH₂)₅, (CH₂)₆, besonders bevorzugt (CH₂)₃ und (CH₂)₄,

- n: - 1 bis 6, vorzugsweise 3 bis 6, besonders bevorzugt 3 und 4,
- R¹²: - C₁- bis C₈-Alkyl, insbesondere C₁- bis C₄-Alkyl wie Methyl, Ethyl, n-Propyl und n-Butyl
- Benzyl,
mit der Maßgabe, daß R¹⁰ nicht Wasserstoff, Methyl, Ethyl, Propyl oder Cyclohexyl bedeutet, wenn R⁷, R⁸, R⁹, R¹¹ Wasserstoff ist, daß R⁷ nicht Methyl bedeutet, wenn R⁸ Methyl oder Phenyl und R⁹, R¹⁰, R¹¹ Wasserstoff ist, daß R⁷ nicht Methyl bedeutet, wenn R⁸, R⁹, R¹⁰, R¹¹ Wasserstoff und R¹² Ethyl ist, daß R⁷ nicht Phenyl oder Hexyl bedeutet, wenn R⁸, R⁹, R¹⁰, R¹¹ Wasserstoff ist, daß R⁹ nicht Methyl bedeutet, wenn R⁷, R⁸, R¹⁰, R¹¹ Wasserstoff ist, daß R¹⁰, R¹¹ nicht Methyl bedeutet, wenn R⁷, R⁸, R⁹ Wasserstoff ist, daß R¹² nicht Methyl ist, wenn R⁸ Phenyl ist und R⁷, R⁹, R¹⁰, R¹¹ Wasserstoff ist, und daß R⁷ und R¹⁰ gemeinsam nicht ein unsubstituiertes Cycloalkyl bedeuten, wenn R⁸, R⁹, R¹¹ Wasserstoff ist.

Die erfindungsgemäßen Verbindungen I und IV dienen als Ausgangsprodukte u.a. für Riechstoffe, Pflanzenschutzmittel und Polymere.

### Beispiele

### Beispiel 1

### Elektrosynthese von 2,3-Epoxy-1-methoxyoctan

Oct-1-en-3-ol wird in der wie folgt beschriebenen Elektrolysezelle unter den angegebenen Bedingungen oxidiert.
- Apparatur:: ungeteilte Zelle mit 9 bipolaren Elektroden
- Anode:: Graphit
- Elektrolyt:: 62,5 g (488 mmol) Oct-1-en-3-ol, 15 g NaBr, 12,5 g H₂O, 535 g Methanol
- Kathode:: Graphit
- Stromdichte:: 6,3 A/dm²
- Elektrolysetemperatur:: 60°C

Die Elektrolyse wird mit 4,75 F/mol Oct-1-en-3-ol durchgeführt. Der Elektrolyt wird während der Elektrolyse mit 20 l/h durch die Zelle gepumpt. Nach Beendigung der Elektrolyse wird das Methanol bei Normaldruck abdestilliert und das ausgefallene Leitsalz abfiltriert. Nach Reindestillation im Vakuum erhält man neben 5,4 g (42 mmol) nicht umgesetztem Oct-1-en-3-ol 28,6 g (37 %) 2,3-Epoxy-1-methoxyoctan als cis, trans-Gemisch; Sdp. 92°C/22 mbar.

### Beispiel 2

### Elektrosynthese von 1,2-Epoxy-3-methoxy-3-methylbutan

3-Methyl-2-buten-1-ol wird unter den in Beispiel 1 beschriebenen Bedingungen elektrooxidiert.
- Elektrolyt:: 62,5 g (727 mmol) 3-Methyl-2-buten-1-ol, 15 g NaBr und 547,5 g Methanol

Die Elektrolyse wird mit 4,4 F/mol Prenol durchgeführt. Der Elektrolyt wird während der Elektrolyse mit 20 l/h durch die Zelle gepumpt. Die Aufarbeitung des Elektrolyseaustrags erfolgt wie in Beispiel 1 beschrieben. Durch Reindestillation bei Normaldruck werden 2,5 g (29 mmol) nicht umgesetztes Edukt und 46,9 g (56 %) 1,2-Epoxy-3-methoxy-3-methylbutan isoliert; Sdp. 115 bis 120°C/1013 mbar.

### Beispiel 3

### Elektrosynthese von 1,2-Epoxy-3-methoxybutan

Crotylalkohol wird unter den in Beispiel 1 beschriebenen Bedingungen elektrooxidiert.
- Elektrolyt:: 62.5 g (868 mmol) Crotylalkohol, 15 g NaBr und 547,5 g Methanol

Elektrolyse mit 5 F/mol Crotylalkohol. Der Elektrolyt wird während der Elektrolyse mit 20 l/h durch die Zelle gepumpt. Die Aufarbeitung erfolgt wie in Beispiel 1 beschrieben. Durch Reindestillation bei Normaldruck wird neben 8,5 g (118 mmol) nicht umgesetztem Crotylalkohol 1,2-Epoxy-3-methoxybutan in einer Ausbeute von 58 % (51,2 g) ; Sdp. 105 bis 110°C/1013 mbar erhalten.

### Beispiel 4

### Elektrosynthese von 1,2-Epoxy-3-methoxy-3-phenylpropan

3-Phenyl-2-propen-1-ol wird unter den in Beispiel 1 beschriebenen Bedingungen in einer ungeteilten Zelle mit 11 bipolaren Elektroden oxidiert.
- Elektrolyt:: 300 g (2,239 mol) 3-Phenyl-2-propen-1-ol, 60 g NaBr und 2 640 g Methanol
- Stromdichte:: 3,4 A/dm²

Die Elektrolyse wird mit 5 F/mol Zimtalkohol durchgeführt. Der Elektrolyt wird während der Elektrolyse mit 200 l/h durch die Zelle gepumpt. Die Aufarbeitung erfolgt wie in Beispiel 1 beschrieben. Durch Reindestillation im Vak. werden 17,5 g (58 mmol) Edukt zurückgewonnen und 187,5 g (51 %) 1,2-Epoxy-3-methoxy-3-phenylpropan; Sdp.: 86°C/3 mbar isoliert.

### Beispiel 5

### Elektrosynthese von 2,3-Epoxy-1-methoxynonan

Non-1-en-3-ol wird unter den in Beispiel 1 beschriebenen Bedingungen in einer ungeteilten Zelle mit 11 bipolaren Elektroden oxidiert.
- Elektrolyt:: 300 g (2.113 mol) Non-1-en-3-ol, 60 g NaBr, 60 g Wasser und 2580 g Methanol
- Stromdichte:: 3,4 A/dm²

Die Elektrolyse wird mit 6,75 F/mol Non-1-en-3-ol durchgeführt. Der Elektrolyt wird während der Elektrolyse mit 200 l/h durch die Zelle gepumpt. Nach Aufarbeitung und Destillation im Vak. werden 15,9 g (112 mmol) Non-1-en-3-ol zurückisoliert und 178,6 g (49 %) 2,3-Epoxy-1-methoxynonan ; Sdp.: 85 bis 88°C/8 mbar erhalten.

### Beispiel 6

### Elektrosynthese von 4,5-Epoxy-6-methoxydecan

Dec-5-en-4-ol wird unter den in Beispiel 1 angegebenen Bedingungen elektrooxidiert.
- Elektrolyt:: 62,5 g (401 mmol) Dec-5-en-4-ol, 15 g NaBr, 12,5 g Wasser und 535 g Methanol

Die Elektrolyse wird mit 4 F/mol Dec-5-en-4-ol durchgeführt. Der Elektrolyt wird während der Elektrolyse mit 20 l/h durch die Zelle gepumpt. Nach Aufarbeitung und Destillation werden 1,4 g (22 mmol) Dec-5-en-4-ol zurückgewonnen und 21,0 g (28 %) 4,5-Epoxy-6-methoxydecan; Sdp.: 50 bis 52°C/1 mbar erhalten.

### Beispiel 7

### Elektrosynthese von 1,2-Epoxy-3-methoxy-3-phenylcyclohexan

3-Phenylcyclohex-2-en-1-ol wird unter den in Beispiel 1 beschriebenen Bedingungen elektrooxidiert.
- Elektrolyt:: 40 g (230 mmol) 3-Phenylcyclohex-2-en-1-ol, 15 g NaBr, 12,5 g Wasser und 535 g Methanol

Die Elektrolyse wird mit 5 F/mol 3-Phenylcyclohex-2-en-1-ol durchgeführt. Der Elektrolyt wird während der Elektrolyse mit 20 l/h durch die Zelle gepumpt. Nach Aufarbeitung und Destillation werden 5,4 g (31 mmol) 3-Phenylcyclohex-2-en-1-ol und 11,8 g (25 %) 1,2-Epoxy-3-methoxy-3-phenylcyclohexan; Sdp.: 120 bis 125°C/2 mbar erhalten.

### Beispiel 8

### Elektrosynthese von 1-Cyclohexyl-1,2-epoxy-3-methoxypropan

1-Cyclohexylprop-2-en-1-ol wird unter den in Beispiel 1 beschriebenen Bedingungen elektrooxidiert.
- Elektrolyt:: 187,5 g (1,339 mol) 1-Cyclohexyl-prop-2-en-1-ol, 45 g NaBr, 37,5 g Wasser und 1605 g Methanol

Die Elektrolyse wird mit 4,4 F/Mol 1-Cyclohexylprop-2-en-1-ol durchgeführt. Der Elektrolyt wird während der Elektrolyse mit 20 l/h durch die Zelle gepumpt. Nach üblicher Aufarbeitung werden 11,4 g (61 mmol) nicht umgesetztes Edukt und 94,9 g (42 %) 1-Cyclohexyl-1,2-epoxy-3-methoxypropan; Sdp.: 75°C/5 mbar erhalten.

### Beispiel 9

### Elektrosynthese von 2,3-Epoxy-4-methoxy-1-phenylbutan

3-Hydroxy-4-phenylbut-1-en wird unter den in Beispiel 1 beschriebenen Bedingungen elektrooxidiert.
- Elektrolyt:: 31,5 g (213 mmol) 3-Hydroxy-4-phenylbut-1-en, 8 g NaBr, 6 g H₂O und 355 g Methanol

Die Elektrolyse wird mit 9 F/mol 3-Hydroxy-4-phenylbut-1-en durchgeführt. Der Elektrolyt wird während der Elektrolyse mit 13 l/h durch die Zelle gepumpt. Nach Aufarbeitung und Destillation werden 3,7 g nicht umgesetztes Edukt und 10,7 g (28 %) 2,3-Epoxy-4-methoxy-1-phenylbutan Sdp. 58-63°C/5 mbar isoliert.

## Patentansprüche

1. Verfahren zur Herstellung von Epoxiden der allgemeinen Formel I 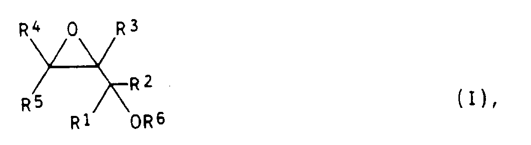 in der die Substituenten
R¹, R², R³, R⁴, R⁵ unabhängig voneinander Wasserstoff, C₁- bis C₂₀-Alkyl, C₃- bis C₁₂-Cycloalkyl, C₄- bis C₂₀-Cycloalkyl-alkyl, C₁- bis C₂₀-Hydroxyalkyl, einen Heterocyclus, gegebenenfalls durch C₁- bis C₈-Alkyl, C₁- bis C₈-Alkoxy, Halogen, C₁- bis C₄-Halogenalkyl, C₁- bis C₄-Halogenalkoxy, Phenyl, Phenoxy, Halogenphenyl, Halogenphenoxy, Carboxy, C₂- bis C₈- Alkoxycarbonyl oder Cyano substituiertes Aryl oder C₇- bis C₂₀-Arylalkyl, oder R¹ und R² oder R¹ und R³ oder R¹ und R⁴ oder R³ und R⁴ oder R⁴ und R⁵ gemeinsam eine gegebenenfalls durch C₁- bis C₈-Alkyl, C₁- bis C₈-Alkoxy und oder Halogen ein- bis zweifach substituierte (CH₂)ₙ-Gruppe, in der n für 1 bis 10 steht und
R⁶ C₁- bis C₈-Alkyl oder Benzyl
bedeuten, dadurch gekennzeichnet, daß man Allylalkohole der allgemeinen Formel II 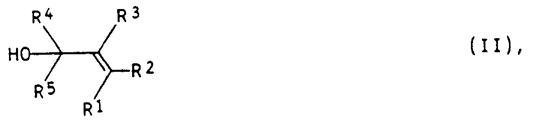 in der R¹, R², R³, R⁴ und R⁵ die obengenannte Bedeutung haben, in Gegenwart eines Alkanols der allgemeinen Formel III
R⁶-OH (III),
in der R⁶ die obengenannte Bedeutung hat, bei Temperaturen von (-20) bis 150°C
in Gegenwart eines Hilfselektrolyten elektrochemisch oxidiert.

2. Verfahren zur Herstellung von Epoxiden der allgemeinen Formel I nach Anspruch 1, dadurch gekennzeichnet, daß man die Umsetzungen bei Temperaturen zwischen 0 und 100°C durchführt.

3. Verfahren nach Anspruch 1, dadurch gekennzeichnet, daß man für die elektrochemische Oxidation einen Elektrolyten der Zusammensetzung
1 bis 49 Gew.% Allylalkohol der Formel II,
50 bis 98,9 Gew.% eines Alkanols der Formel III und
0,1 bis 5 Gew.% eines Hilfselektrolyten
verwendet.

4. Verfahren nach Anspruch 1, dadurch gekennzeichnet, daß man die elektrochemische Oxidation an Graphitelektroden durchführt.

5. Verfahren nach Anspruch 1, dadurch gekennzeichnet, daß man die elektrochemische Oxidation bei Stromdichten von 0,5 bis 25 A/dm² durchführt.

6. Verfahren nach Anspruch 1, dadurch gekennzeichnet, daß man als Hilfselektrolyte Jodide oder Bromide einsetzt.

7. α-Alkoxyepoxide der allgemeinen Formel IV 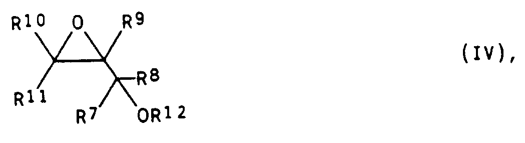 in der die Substituenten folgende Bedeutung haben:
R⁷, R⁸, R⁹, R¹⁰, R¹¹ Wasserstoff, C₁- bis C₂₀-Alkyl, C₃- bis C₁₂-Cycloalkyl, C₄- bis C₂₀-Cycloalkyl-alkyl, C₁- bis C₂₀-Hydroxyalkyl, einen Heterocyclus, gegebenenfalls durch C₁- bis C₈-Alkyl, C₁- bis C₈-Alkoxy, Halogen, C₁- bis C₄-Halogenalkyl, C₁- bis C₄-Halogenalkoxy, Phenyl, Phenoxy, Halogenphenyl, Halogenphenoxy, Carboxy, C₂- bis C₈- Alkoxycarbonyl oder Cyano substituiertes Aryl oder C₇- bis C₂₀-Arylalkyl, oder R⁷ und R⁸ oder R⁷ und R⁹ oder R⁷ und R¹⁰ oder R⁹ und R¹⁰ oder R¹⁰ und R¹¹ gemeinsam eine gegebenenfalls durch C₁- bis C₈-Alkyl, C₁- bis C₈-Alkoxy und oder Halogen ein- bis zweifach substituierte (CH₂)ₙ-Gruppe, in der n für 1 bis 10 und
R¹² C₁- bis C₈-Alkyl oder Benzyl
bedeuten,
mit der Maßgabe, daß R¹⁰ nicht Wasserstoff, Methyl, Ethyl, Propyl oder Cyclohexyl bedeutet, wenn R⁷, R⁸, R⁹, R¹¹ Wasserstoff ist, daß R⁷ nicht Methyl bedeutet, wenn R⁸ Methyl oder Phenyl und R⁹, R¹⁰, R¹¹ Wasserstoff ist, daß R⁷ nicht Methyl bedeutet, wenn R⁸, R⁹, R¹⁰, R¹¹ Wasserstoff und R¹² Ethyl ist, daß R⁷ nicht Phenyl oder Hexyl bedeutet, wenn R⁸, R⁹, R¹⁰, R¹¹ Wasserstoff ist, daß R⁹ nicht Methyl bedeutet, wenn R⁷, R⁸, R¹⁰, R¹¹ Wasserstoff ist, daß R¹⁰, R¹¹ nicht Methyl bedeutet, wenn R⁷, R⁸, R⁹ Wasserstoff ist, daß R¹² nicht Methyl ist, wenn R⁸ Phenyl ist und R⁷, R⁹, R¹⁰, R¹¹ Wasserstoff ist, und daß R⁷ und R¹⁰ gemeinsam nicht ein unsubstituiertes Cycloalkyl bedeuten, wenn R⁸, R⁹, R¹¹ Wasserstoff ist.

## Claims

1. A process for preparing epoxides of the general formula I 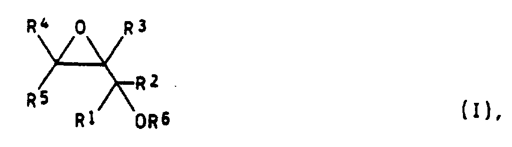 where R¹, R², R³, R⁴ and R⁵ are each, independently of one another, hydrogen, C₁-C₂₀-alkyl, C₃-C₁₂-cycloalkyl, C₄-C₂₀-cycloalkylalkyl, C₁-C₂₀-hydroxyalkyl, a heterocycle, unsubstituted or C₁-C₈-alkyl-, C₁-C₈-alkoxy-, halogen-, C₁-C₄-haloalkyl-, C₁-C₄-haloalkoxy-, phenyl-, phenoxy-, halophenyl-, halophenoxy-, carboxyl-, C₂-C₈-alkoxycarbonyl- or cyano-substituted aryl or C₇-C₂₀-arylalkyl, or R¹ and R² or R¹ and R³ or R¹ and R⁴ or R³ and R⁴ or R⁴ and R⁵ together are a (CH₂)ₙ group in which n is 1-10 and which is unsubstituted or mono- or disubstituted by C₁-C₈-alkyl, C₁-C₈-alkoxy and/or halogen, and R⁶ is C₁-C₈-alkyl or benzyl,
which comprises electrochemical oxidation of allyl alcohols of the general formula II 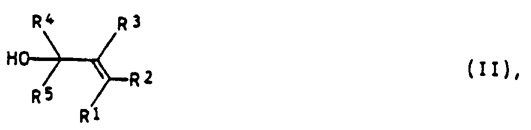 where R¹, R², R³, R⁴ and R⁵ have the abovementioned meaning, in the presence of an alkanol of the general formula III
R⁶-OH (III),
where R⁶ has the abovementioned meaning, at from -20 to 150°C, in the presence of an auxiliary electrolyte.

2. A process for preparing epoxides of the general formula I as claimed in claim 1, wherein the reactions are carried out at from 0 to 100°C.

3. A process as claimed in claim 1, wherein an electrolyte of the composition 1 - 49% by weight of allyl alcohol of the formula II, 50 - 98.9% by weight of an alkanol of the formula III and 0.1 - 5% by weight of an auxiliary electrolyte is used for the electrochemical oxidation.

4. A process as claimed in claim 1, wherein the electrochemical oxidation is carried out on graphite electrodes.

5. A process as claimed in claim 1, wherein the electrochemical oxidation is carried out with current densities of 0.5 - 25 A/dm².

6. A process as claimed in claim 1, wherein iodides or bromides are used as auxiliary electrolytes.

7. An α-hydroxy epoxide or α-alkoxy epoxide of the general formula IV 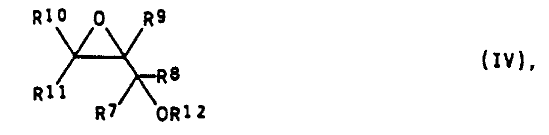 where R⁷, R⁸, R⁹, R¹⁰ and R¹¹ are each hydrogen, C₁-C₂₀-alkyl, C₃-C₁₂-cycloalkyl, C₄-C₂₀-cycloalkylalkyl, C₁-C₂₀-hydroxyalkyl, a heterocycle, unsubstituted or C₁-C₈-alkyl-, C₁-C₈-alkoxy-, halogen-, C₁-C₄-haloalkyl-, C₁-C₄-haloalkoxy-, phenyl-, phenoxy-, halophenyl-, halophenoxy-, carboxyl-, C₂-C₈-alkoxycarbonyl- or cyano-substituted aryl or C₇-C₂₀-arylalkyl, or R⁷ and R⁸ or R⁷ and R⁹ or R⁷ and R¹⁰ or R⁹ and R¹⁰ or R¹⁰ and R¹¹ together are a (CH₂)ₙ group in which n is 1-10 and which is unsubstituted or mono- or disubstituted by C₁-C₈-alkyl, C₁-C₈-alkoxy and/or halogen, and
R¹² is C₁-C₈-alkyl or benzyl,
with the proviso that R¹⁰ is not hydrogen, methyl, ethyl, propyl or cyclohexyl when R⁷, R⁸, R⁹, R¹¹ is hydrogen, that R⁷ is not methyl when R⁸ is methyl or phenyl and R⁹, R¹⁰, R¹¹ is hydrogen, that R⁷ is not methyl when R⁸, R⁹, R¹⁰, R¹¹ is hydrogen and R¹² is ethyl, that R⁷ is not phenyl or hexyl when R⁸, R⁹, R¹⁰, R¹¹ is hydrogen, that R⁹ is not methyl when R⁷, R⁸, R¹⁰, R¹¹ is hydrogen, that R¹⁰, R¹¹ is not methyl when R⁷, R⁸, R⁹ is hydrogen, that R¹² is not methyl when R⁸ is phenyl and R⁷, R⁹, R¹⁰, R¹¹ is hydrogen, and that R⁷ and R¹⁰ together are not an unsubstituted cycloalkyl when R⁸, R⁹, R¹¹ is hydrogen.

## Revendications

1. Procédé de préparation d'époxydes de la formule générale I : 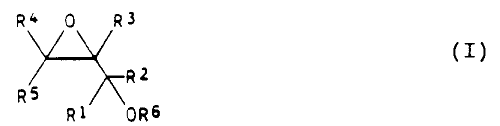 dans laquelle les symboles
R¹, R², R³, R⁴, R⁵, représentent, chacun indépendamment l'un de l'autre, un atome d'hydrogène, un radical alkyle en C₁-C₂₀, cycloalkyle en C₃-C₁₂, cycloalkyle(C₄-C₂₀)alkyle, hydroxyalkyle en C₁-C₂₀, un noyau hétérocyclique, un radical aryle(C₇-C₂₀)alkyle, ou aryle, éventuellement substitué par des radicaux alkyle en C₁-C₈, alcoxy en C₁-C₈, des atomes d'halogène, des radicaux halogénoalkyle en C₁-C₄, halogénoalcoxy en C₁-C₄, phényle, phénoxy, halogénophényle, halogénophénoxy, carboxyle, alcoxy(C₂-C₈)carbonyle ou cyano, ou bien R¹ et R², ou R¹ et R³, ou R¹ et R⁴, ou R³ et R⁴, ou R⁴ et R⁵, forment ensemble un radical (CH₂)ₙ éventuellement mono ou bisubstitué par des radicaux alkyle en C₁-C₈, alcoxy en C₁-C₈ et/ou des atomes d'halogène, radical dans lequel n a une valeur qui varie de 1 à 10, et
R⁶ représente un radical alkyle en C₁-C₈ ou benzyle,
caractérisé en ce que l'on oxyde des alcools allyliques de la formule générale II : 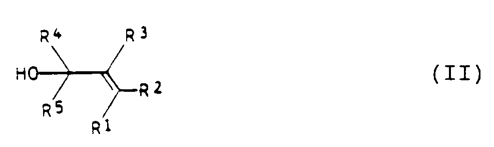 dans laquelle
R¹, R², R³, R⁴ et R⁵ possèdent les significations qui leur ont été précédemment attribuées, en présence d'un alcool de la formule générale III :
R⁶-OH (III)
dans laquelle
R⁶ possède les significations qui lui ont été précédemment attribuées, à des températures de (-20°C) à 150°C,
en présence d'un électrolyte auxiliaire, par voie électrochimique.

2. Procédé de préparation d'époxydes de la formule générale I suivant la revendication 1, caractérisé en ce que l'on entreprend les réactions à des températures qui varient de 0 à 100°C.

3. Procédé suivant la revendication 1, caractérisé en ce que l'on utilise, en vue de l'oxydation électrochimique, un électrolyte de la composition suivante :
1 à 49% en poids d'alcool allylique de la formule II
50 à 98,9% en poids d'un alcanol de la formule III, et
0,1 à 5% en poids d'un électrolyte auxiliaire.

4. Procédé suivant la revendication 1, caractérisé en ce que l'on entreprend l'oxydation électrochimique sur des électrodes en graphite.

5. Procédé suivant la revendication 1, caractérisé en ce que l'on entreprend l'oxydation électrochimique à des densités de courant de 0,5 à 25 A/dm².

6. Procédé suivant la revendication 1, caractérisé en ce que l'on utilise des iodures ou des bromures à titre d'électrolytes auxiliaires.

7. α-Hydroxyépoxydes et α-alcoxyépoxydes de la formule générale IV : 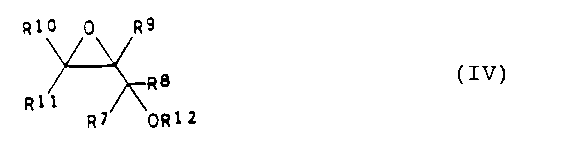 dans laquelle les divers symboles
R⁷, R⁸, R⁹, R¹⁰ et R¹¹ représentent chacun un atome d'hydrogène, un radical alkyle en C₁-C₂₀, cycloalkyle en C₃-C₁₂, cycloalkyle(C₄-C₂₀)alkyle, hydroxyalkyle en C₁-C₂₀, un noyau hétérocyclique, un radical aryle (C₇-C₂₀)alkyle, ou aryle, éventuellement substitué par des radicaux alkyle en C₁-C₈, alcoxy en C₁-C₈, des atomes d'halogène, des radicaux halogénoalkyle en C₁-C₄, halogénoalcoxy en C₁-C₄, phényle, phénoxy, halogénophényle, halogénophénoxy, carboxyle, alcoxy(C₂-C₈)carbonyle ou cyano, ou bien R⁷ et R⁸, ou R⁷ et R⁹, ou R⁷ et R¹⁰, ou R⁹ et R¹⁰, ou R¹⁰ et R¹¹, forment ensemble un radical (CH₂)ₙ éventuellement mono ou bisubstitué par des radicaux alkyle en C₁-C₈, alcoxy en C₁-C₈, et/ou des atomes d'halogène, radical dans lequel n a une valeur qui varie de 1 à 10, et
R¹² représente un radical alkyle en C₁-C₈ ou benzyle,
avec la condition que R¹⁰ ne représente ni atome d'hydrogène, ni radical méthyle, éthyle, propyle ou cyclohexyle, lorsque R⁷, R⁸, R⁹, R¹¹ représentent des atomes d'hydrogène, que R⁷ ne représente pas de radical méthyle lorsque R⁸ représente un groupe méthyle ou phényle et R⁹, R¹⁰, R¹¹ représentent des atomes d'hydrogène, que R⁷ ne représente pas de radical méthyle lorsque R⁸, R⁹, R¹⁰, R¹¹ représentent des atomes d'hydrogène et R¹² représente le radical éthyle, que R⁷ ne représente ni radical phényle, ni radical hexyle lorsque R⁸, R⁹, R¹⁰, R¹¹ représentent des atomes d'hydrogène, que R⁹ ne représente pas de radical méthyle lorsque R⁷, R⁸, R¹⁰, R¹¹ représentent des atomes d'hydrogène, que R¹⁰, R¹¹ ne représentent pas de radicaux méthyle lorsque R⁷, R⁸, R⁹ représentent des atomes d'hydrogène, que R¹² ne représente pas de radical méthyle lorsque R⁸ représente un radical phényle et R⁷, R⁹, R¹⁰, R¹¹ représentent des atomes d'hydrogène et que R⁷ et R¹⁰ ensemble ne représentent pas de radical cycloalkyle non substitué lorsque R⁸, R⁹, R¹¹ représentent des atomes d'hydrogène.
